Europäisches Patentamt

European Patent Office

Office européen des brevets

(11) Numéro de publication: **0 228 359**
A2

# (12) DEMANDE DE BREVET EUROPEEN

(21) Numéro de dépôt: 86870203.6

(22) Date de dépôt: 31.12.86

(51) Int. Cl.⁴: **G 01 N 33/543**
G 01 N 33/52

(30) Priorité: 02.01.86 LU 86239

(43) Date de publication de la demande:
08.07.87 Bulletin 87/28

(84) Etats contractants désignés:
AT BE CH DE ES FR GB GR IT LI NL SE

(71) Demandeur: **LABORATOIRES SANDERS-PROBEL, S.A.**
**47-51 rue Henri Wafelaerts Saint-Gilles**
**B-1060 Bruxelles (BE)**

(72) Inventeur: **Maggipinto, Gianni**
**152 rue du Village**
**B-4420 Rocourt (BE)**

(74) Mandataire: **Vigneron, Jean**
**Cabinet VIGNERON 30 avenue Eugène Godaux**
**B-1150 Bruxelles (BE)**

(54) **Méthode de détection d'agents réactifs et moyens de mise en oeuvre de cette méthode.**

(57) Méthode de détection d'agents réactifs dans laquelle un agent détecteur, capable de s'associer à un agent à détecter, est fixé sur un support, ce support est mis en contact avec un milieu à analyser, un agent magnétisable est associé à l'association de l'agent à détecter et l'agent détecteur fixé sur le support, ou un agent magnétisable préalablement associé à l'agent détecteur fixé sur le support est déplacé par l'association de l'agent à détecter et l'agent détecteur, et la présence, respectivement l'absence de l'agent magnétisable est détectée par un moyen approprié.

EP 0 228 359 A2

## Description

Méthode de détection d'agents réactifs et moyens de mise en oeuvre de cette méthode.

La présente invention a pour objet une nouvelle méthode de détection d'agents réactifs quelconques tels que des agents chimiques ou biologiques.

La méthode selon l'invention est particulièrement utile pour la détection et le dosage d'agents biologiques du type antigènes et anticorps, mais elle peut trouver des applications beaucoup plus générales, notamment pour la détection d'agents chimiques dans les domaines de l'industrie chimique et de la sécurité.

La méthode selon l'invention implique la fixation ("coating" ou "coatage") sur un support d'un réactif spécifique de (c'est-à-dire capable de s'associer à ) l'agent à détecter et la mise en évidence d'une association (une réaction) entre ledit réactif et l'agent à détecter, par un moyen particulièrement pratique à l'usage.

Les techniques habituellement utilisées pour mettre en évidence par exemple la réaction Antigène-Anticorps font appel à des conjugués fluorescents (lecture au microscope ou au flow cytomètre), à un traceur radioactif (lecture au compteur de particules) ou aux enzymes (lecture au spectrophotomètres).

Les conjugués fluorescents demandent afin d'être visualisés un support cellulaire ou microsphère, de plus l'appréciation des résultats est qualitative, semi-quantitative (dilution).

Les traceurs radioactifs font appel comme leur nom l'indique à la radioactivité et ce que cela comporte en inconvénients(déchets, précautions de manipulation, locaux adaptés).

Les conjugués enzymatiques quant à eux, bien qu'étant très pratiques et en plein essor, demandent une étape supplémentaire (substrat).

La technique idéale devrait rassembler tous les avantages des précédentes mais aucun des inconvénients.

De même les techniques de détection de certains agents chimiques impliquent le captage de l'agent concerné par un réactif approprié, après quoi un problème se pose pour la mise en évidence de l'agent chimique ainsi capté.

La présente invention propose une technique de mise en évidence d'une association entre un agent à détecter et un réactif détecteur fixé sur un support, par des moyens magnétiques.

La méthode de détection d'agents réactifs selon l'invention implique que :
au moins un agent détecteur capable de s'associer à au moins un agent à détecter, est fixé sur un support ;
le support ainsi préparé est mis en contact avec un milieu à analyser ;
une association entre un agent à détecter et un agent détecteur est mis en évidence sur le support ;
et se caractérise par le fait qu'un agent magnétisable est associé à l'association de l'agent à détecter et l'agent détecteur fixé sur le support ou qu'un agent magnétisable préalablement associé à l'agent détecteur fixé sur le support est déplacé par l'association de l'agent à détecter et l'agent détecteur, et que la présence, respectivement l'absence de l'agent magnétisable est détectée par un moyen approprié.

Selon un mode réalisation particulièrement avantageux de l'invention il est possible de disposer l'agent détecteur en piste(s) ou en bande(s) sur le support, de manière à permettre plus facilement la réalisation en série de la méthode selon l'invention.

Un mode de réalisation particulièrement préféré de la méthode selon l'invention prévoit l'utilisation d'un support qui est lui-même sous forme de bande, en particulier sous forme de bandes plastiques.

La mise en oeuvre de la méthode selon l'invention peut procéder de diverses façons.

Il est par exemple possible de mettre différentes portions d'une bande munie d'un (même) agent détecteur en contact avec différents milieux à analyser et de réaliser ainsi une méthode avantageuse d'analyses en série.

Il est d'autre part également possible, par exemple, d'utiliser une bande sur laquelle sont fixés, de manière séquentielle, différents agents détecteurs, ce qui permet de réaliser une série de tests différents sur un même milieu à analyser.

Selon une autre particularité préférée de l'invention, la présence respectivement l'absence de l'agent magnétisable, consécutive de l'association de l'agent à détecter avec l'agent détecteur fixé sur le support peut être mise en évidence par magnétisation du support réactionnel et par lecture au moyen d'un lecteur approprié.

Dans le cas d'une bande plastique, il est notamment possible, de manière particulièrement avantageuse, de mettre en oeuvre des systèmes de magnétisation ("enregistrement") et de lecture classiques du type "tape recorder" audio ou vidéo.

Selon l'une alternative de la méthode de l'invention, on met en évidence l'absence de l'agent magnétisable consécutive à l'association de l'agent à détecter et de l'agent détecteur.

Cette alternative peut par exemple résulter de l'utilisation d'un support sur lequel est fixé un agent détecteur auquel est associé un agent magnétisable qui présente une affinité vis-à-vis de l'agent détecteur plus faible que celle de l'agent à détecter vis-à-vis de l'agent détecteur.

La présence d'agent à détecter dans le milieu analysé impliquera alors le déplacement de l'agent magnétisable par l'agent à détecter, ce qui se manifestera à la "lecture" par une absence de magnétisation à cet endroit.

Un mode de réalisation particulièrement préféré de la méthode selon l'invention concerne la détection des antigènes respectivement anticorps. Pour ce faire, on utilise un support sur lequel sont fixés en tant qu'agents détecteurs, des antigènes respectivement des anticorps tandis que les agents réactifs à détecter consistent en les anticorps respectivement les antigènes spécifiques correspondants.

Dans ce mode de réalisation de l'invention, il peut de manière préférée être prévu de "révéler" (mettre en évidence) l'association entre l'antigène (respectivement l'anticorps) fixé sur le support et l'anticorps (respectivement l'antigène) à détecter, par réaction avec un second anticorps dans lequel est incorporé ou auquel est conjugué un agent magnétisable.

Dans ce mode de réalisation, on peut donc par exemple coater sur une bande un antigène (ou anticorps), faire réagir cette band avec l'anticorps (ou l'antigène) correspondant et révéler cette réaction à l'aide d'un second anticorps dans lequel on a incorporé des particules magnétiques ou conjugué par toute autre méthode à des particules magnétisables. La lecture se fait après enregistrement (magnétisation) à une fréquence donnée, sur un lecteur de bande appelé couramment "tape" et de visualiser sur oscilloscope.La quantité de particules magnétisées est proportionnelle à la quantité de complexes Antigène/Anticorps, donc quantifiable.

L'invention concerne également les moyens à mettre en oeuvre pour la réalisation de la méthode selon l'invention.Ainsi, l'invention concerne notamment un support coaté au moyen d'antigènes respectivement d'anticorps caractérisé par le fait que le support se présente sous la forme d'une bande plastique.L'invention a donc également pour objet toute bande plastique munie à sa surface d'une couche (coating) d'antigènes, respectivement d'anticorps.

Cette bande plastique peut selon une réalisation préférée de l'invention notamment être constituée de plages alternativement munies d'une couche magnétisable et d'une couche d'antigènes respectivement anticorps.

Une telle réalisation permet le codage de la bande en fonction des tests effectués.

La bande plastique peut également selon une autre réalisation préférée de l'invention être constituée de plages successives munies d'une couche de différents antigènes, respectivement anticorps, ce qui permet au moyen d'une "bande d'analyse" d'effectuer différents tests sur un même milieu à analyser.

L'invention a d'autre part également pour objet des anticorps dans lesquels sont incorporés ou auxquels sont conjugués des agents magnétisables, sous une forme destinée à la méthode selon l'invention.

Sans déchets radioactifs, ni étape supplémentaire, la méthode selon l'invention est quantitative et permet une lecture intégrée, c'est-à-dire qu'une surface est balayée par une cellule de lecture donnant un résultat final dépendant de la vitesse de passage et de la longueur de la bande support réactionnel, permettant une meilleure descrimination positif/négatif (seuil de sensibilité très abaissé). De plus, toutes les variantes basées sur l'association d'agents réactifs (réactions compétitives, sandwich, reverse, etc. . .) sont applicables. De même qu'une bande peut être coatée par des substances différentes pour le screening en sérologie, ou en application directe en bactérie.

Les techniques de coatage décrites par Engvall et modifiées par Voller, consistant à mettre l'antigène en tampon basique et laisser réagir cette solution avec le support solide sur lequel l'antigène va s'adsorber préférentiellement par sa partie hydrophobe (COOH) peuvent s'appliquer à tout support plastique (PVC, polystirène, nylon . . .) Singer et coll. ont décrit en 1961 une méthode de couplage de la ferritine à un antigène ou anticorps pour une application au microscope électronique (méthode de densité électronique). Une application de cette méthode consiste à coupler des particules magnétiques à un anticorps monoclonal, et de séparer par aimantation les complexes Antigène/Anticorps formés.

## Revendications

1.- Méthode de détection d'agents réactifs dans laquelle :
au moins un agent détecteur, capable de s'associer à au moins un agent à détecter, est fixé sur un support ;
le support ainsi préparé est mis en contact avec un milieu à analyser ;
une association entre un agent à détecter et un agent détecteur est mis en évidence sur le support ;
caractérisée par le fait qu'un agent magnétisable est associé à l'association de l'agent à détecter et l'agent détecteur fixé sur le support, ou qu'un agent magnétisable préalablement associé à l'agent détecteur fixé sur le support est déplacé par l'association de l'agent à détecter et l'agent détecteur, et que la présence, respectivement l'absence de l'agent magnétisable est détectée par un moyen approprié.

2.- Méthode de détection d'agents réactifs selon la revendication 1, caractérisée par le fait que l'agent détecteur est disposé en piste(s) ou en bande(s) sur le support.

3.- Méthode de détection d'agents réactifs selon la revendication 1, caractérisée par le fait que le support sur lequel est fixé l'agent détecteur se présente sous la forme d'une bande.

4.- Méthode de détection d'agents réactifs selon la revendication 3, caractérisée par le fait que différentes portions de ladite bande de support sont mis en contact avec différents milieux à analyser.

5.- Méthode de détection d'agents réactifs selon la revendication 3, caractérisée par le fait que différents agents détecteurs sont fixés sur différentes portions de la bande support, de façon à détecter différents agents réactifs dans un milieu à analyser.

6.- Méthode de détection d'agents réactifs selon l'une ou l'autre des revendications 3 à 5, caractérisée par le fait que la présence, respectivement l'absence de l'agent magnétisable est mise en évidence par la magnétisation de la bande et par sa lecture sur un lecteur de bandes magnétiques.

7.- Méthode de détection d'agents réactifs selon l'une ou l'autre des revendications précédentes caractérisée par le fait que l'agent détecteur fixé sur le support est associé à un agent magnétisable ayant une affinité moins grande pour l'agent détecteur que l'agent réactif à détecter.

8.- Méthode de détection d'agents réactifs selon l'une ou l'autre des revendications précédentes, caractérisée par le fait que l'agent détecteur fixé sur le support est un anticorps respectivement un antigène, tandis que l'agent réactif à détecter est l'antigène respectivement l'anticorps spécifique correspondant.

9.- Méthode de détection d'agents réactifs selon la revendication 8, caractérisée par le fait que l'association entre un anticorps (respectivement antigène) fixé sur le support et un antigène (respectivement anticorps) à détecter est révélée par réaction d'un second anticorps dans lequel est incorporé ou auquel est conjugué un agent magnétisable.

10.- Support coaté au moyen d'antigènes respectivement d'anticorps caractérisé par le fait que le support se présente sous la forme d'une bande plastique.

11.-Bande plastique caractérisée par le fait qu'elle est munie d'une couche (coating) d'antigènes respectivement anticorps fixés à sa surface.

12.- Bande plastique selon la revendication 11, caractérisée par le fait qu'elle est constituée de plages alternativement munies d'une couche magnétisable et d'une couche d'antigènes respectivement anticorps.

13.- Bande plastique selon la revendication 11, caractérisée par le fait qu'elle est constituée de plages successives munies d'une couche de différents antigènes respectivement anticorps.

14.- Anticorps dans lequel est incorporé ou auquel est conjugué un agent magnétisable destiné à la méthode selon la revendication 9.